# EUROPEAN PATENT APPLICATION

(11) **EP 1 911 842 A1**
(43) Date of publication of application: **16.04.2008**
(21) Application number: 06747033.6
(22) Date of filing: 31.05.2006
(51) Int. Cl.: C12N 15/09, A01K 67/027, C12N 1/02, C12N 5/10, C12Q 1/68

(54) **EXPRESSION VECTOR HAVING PROMOTER SEQUENCE OF Vasa HOMOLOGUE GENE DERIVED FROM MAMMAL AND USE THEREOF**

(30) Priority: 01.06.2005 JP 2005161279
(71) Applicant: Mitsubishi Chemical Corporation, Minato-ku Tokyo 108-0014 (JP)
(72) Inventor: NOCE, Toshiaki, MITSUBISHI KAGAKU INST. OF LIFE SCIENCES, Machida-shi, Tokyo 1948511 (JP); ABE, Kuniya, c/o RIKEN, Tsukuba Institute, Tsukuba-shi, Ibaraki 3050074 (JP); MISE, Natan, c/o RIKEN, Tsukuba Institute, Tsukuba-shi, Ibaraki 3050074 (JP)
(74) Representative: HOFFMANN EITLE
(86) International application number: PCT/JP2006/310860
(87) International publication number: WO 2006/129696

(57) **Abstract**

It is an object of the present invention to provide a method for selecting cells capable of differentiating germ cells by employing expression of marker genes as an indicator in a simple manner without complicated operations such as homologous recombination. The present invention provides a method for obtaining germ cells which comprises recovering cells capable of differentiating germ cells from a transgenic non-human mammalian animal into which a recombinant expression vector comprising a marker gene under the control of a promoter sequence of a mammalian-derived Vasa homolog gene has been introduced by employing expression of said marker gene as an indicator.

## Description

### Technical Field

The present invention relates to a recombinant expression vector wherein a marker gene is incorporated under the control of a promoter sequence of a mammalian-derived Vasa homolog gene, and a method for obtaining germ cells from a transgenic non-human mammalian animal, ES cells, or tissue stem cells, into which such recombinant expression vector has been introduced.

### Background Art

Genetic analysis involving the use of fruit flies has demonstrated that genes, such as Oskar, Vasa, Tudor, and Nanos, have core functions in the germ cell-determining mechanism (Rongo, C., et al, Development, 121, 2737-2746,1995). These genes are accumulated in the form of polar granules at the time of egg formation, and blastomeres having maternal determinants determine the fate of germ cells. Vasa genes are considered to encode ATP-dependent RNA helicases, and functions thereof are considered to be associated with regulation of translation from mRNAs to proteins (Liang, L., et al, Development, 120, 1201-1211, 1994). Since the mechanisms thereof in charge of enzyme functions are evolutionally strongly conserved, Vasa homolog genes are identified in many multicellular animal species, from planarians to humans. Mouse Vasa homolog genes (Mvh) are registered under the gene name Ddx4 (gene ID: 13206) in the mouse gene databank. The Mvh locus containing the entire transcription unit (exon) is of a length of approximately 53 kb and is located on mouse chromosome 13 between a protein gene (RIKEN cDNA 9130023D20) located about 9.5 kb upstream in the transcription direction and the Tubulin-2 pseudo genes located about 3.2 kb downstream. The conformational characteristics thereof are the same as those of primate Vasa homolog genes.

Expression of Mvh genes is detected in all oogenetic and spermatogenetic germ cell lineages from migratory primordial germ cells during the fetal period to gonadal primordial germ cells and post-meiotic haploid germ cells. Expression specificity is common in all mammalian animal species, including vertebrate animals and, particularly, humans. Expression of Vasa homolog genes exhibits very strict germ cell-specificity among the germ cell-expressing genes that have been identified in the past (Toyooka, Y et al, Mech. Dev., 93, 139-149, 2000).

JP Patent Publication (kokai) No. 2002-65261 A discloses a method for obtaining germ cells comprising culturing an ES cell strain into which a marker gene has been introduced under the control of expressing a Vasa gene or a homologous gene thereof in the presence of a germ cell differentiation promotive factor and selecting cells having the ability to differentiate germ cells by employing expression of the marker gene as an indicator. According to the method of JP Patent Publication (kokai) No. 2002-65261 A, a marker gene is introduced into an ES cell line via homologous recombination under the control of the expression of a Vasa gene that is endogenous in the ES cell or a homologous gene thereof.

Also, Tanaka, M. et al, PNAS, Vol. 98, No. 5, p. 2544-2549, 2001 discloses transgenic *Oryzias latipes* which was produced by introducing a Vasa vector having a green fluorescent protein (GFP) ligated thereto into a Vasa gene promoter of *Oryzias latipes.* According to Tanaka, M. et al, PNAS, Vol. 98, No. 5, p. 2544-2549, 2001, cell lineages before male and female germ cells are formed have been observed; however, separation of germ cells via culture of such cell lineages has not been attempted. Further, there is no report concerning the production of a transgenic mammalian animal into which a Vasa vector has been introduced.

### Disclosure of the Invention

### Object to be Attained by the Invention

It is an object of the present invention to provide a method for selecting cells capable of differentiating germ cells by employing expression of marker genes as an indicator in a simple manner without complicated operations such as homologous recombination.

### Means for Attaining the Object

The present inventors have conducted concentrated studies in order to attain the above object. They prepared a transgenic non-human mammalian animal into which a recombinant expression vector comprising a marker gene under the control of a promoter sequence of a mammalian-derived Vasa homolog gene had been introduced. They then discovered that cells capable of differentiating germ cells could be selected from a transgenic non-human mammalian animal by employing expression of such marker gene as an indicator. The present invention has been completed based on such finding.

Specifically, the present invention, provides the following inventions.
(1) A method for obtaining germ cells which comprises recovering cells capable of differentiating germ cells from a transgenic non-human mammalian animal into which a recombinant expression vector comprising a marker gene under the control of a promoter sequence of a mammalian-derived Vasa homolog gene has been introduced by employing expression of said marker gene as an indicator.
(2) A method for obtaining germ cells which comprises culturing ES cells or tissue stem cells into which a recombinant expression vector comprising a marker gene under the control of a promoter sequence of a mammalian-derived Vasa homolog gene has been introduced and recovering cells capable of differentiating germ cells by employing expression of said marker gene as an indicator.
(3) The method according to (2), wherein the ES cells or tissue stem cells are mammalian- or avian-derived cell lines.
(4) The method according to any of (1) to (3), wherein the promoter sequence of a mammalian-derived Vasa homolog gene comprises at least a nucleotide sequence comprising a region from a site 3 kb upstream of the transcription initiation point to the transcription initiation point.
(5) The method according to any of (1), to (4), wherein the recombinant vector comprises, at a site downstream of the marker gene, a sequence of the 3'-untranslated region (UTR) of a mammalian-derived Vasa homolog gene ligated thereto.
(6) The method according to any of (1) to (5), wherein the marker gene is a fluorescent protein-encoding gene, a drug-resistant gene, or a chemiluminescent enzyme gene.
(7) The method according to any of (1) to (6), wherein the marker gene is a GFP, RFP, BFP, YFP, or lacZ gene.
(8) The method according to any of (1) to (7), wherein the germ cells are germ stem cells.
(9) A recombinant expression vector which comprises a marker gene under the control of a promoter sequence of a mammalian-derived Vasa homolog gene.
(10) The recombinant expression vector according to (9), wherein the promoter sequence of a mammalian-derived Vasa homolog gene comprises at least a nucleotide sequence comprising a region from a site 3 kb upstream of the transcription initiation point to the transcription initiation point
(11) The recombinant expression vector according to (9) or (10), wherein the recombinant vector comprises, at a site downstream of the marker gene, a sequence of the 3'-untranslated region (UTR) of a mammalian-derived Vasa homolog gene ligated thereto.
(12) The recombinant expression vector according to any of (9) to (11), wherein the marker gene is a fluorescent protein-encoding gene, a drug-resistant gene, or a chemiluminescent enzyme gene.
(13) The recombinant expression vector according to any of (9) to (12), wherein the marker gene is a GFP, RFP, BFP, YFP, or lacZ gene.
(14) The recombinant expression vector according to any of (9) to (13), which is used for the method according to any of (1) to (8).
(15) A cell which comprises the recombinant expression vector according to any of (9) to (14).
(16) The cell according to (15), which is an ES cell or a tissue stem cell.
(17) A transgenic non-human mammalian animal into which the recombinant expression vector according to any of (9) to (14) has been introduced.
(18) A method for screening for a germ cell differentiation enhancing factor which comprises administering a test substance to a transgenic non-human mammalian animal into which a recombinant expression vector comprising a marker gene under the control of a promoter sequence of a mammalian-derived Vasa homolog gene has been introduced and selecting a test substance that induces expression of the marker gene.
(19) A method for screening for a germ cell differentiation enhancing factor which comprises culturing an ES cell or a tissue stem cell into which a recombinant expression vector comprising a marker gene under the control of a promoter sequence of a mammalian-derived Vasa homolog gene has been introduced in the presence of a test substance and selecting a test substance that induces expression of the marker gene.
(20) A germ cell differentiation enhancing factor which is obtained by the method according to (18) or (19).
(21) A method for testing toxicity to germ cells which comprises administering a test substance to a transgenic non-human mammalian animal into which a recombinant expression vector comprising a marker gene under the control of a promoter sequence of a mammalian-derived Vasa homolog gene has been introduced and detecting a substance having toxicity to germ cells by employing expression of said marker gene as an indicator.
(22) A method for testing toxicity to germ cells which comprises culturing an ES cell or a tissue stem cell into which a recombinant expression vector comprising a marker gene under the control of a promoter sequence of a mammalian-derived Vasa homolog gene has been introduced in the presence of a test substance and detecting a substance having toxicity to germ cells by employing the number of cells in which expression of the marker gene is induced as an indicator.

### Best Modes for Carrying out the Invention

Hereafter, the implementation methods and embodiments of the present invention are described in detail.

### (1) Recombinant expression vector of the present invention

In order to identify differentiation from ES cells into primordial germ cells, use of traits that are not expressed in ES cells but are expressed specifically in differentiated germ cells as indicators is necessary. Germ cell-specific Vasa homolog genes are specific for late primordial germ cells that reside in the gonad, but expression of such genes is not observed in undifferentiated early embryo cells at earlier stages or ES cells. Thus, in the present invention, expression of mammalian-derived Vasa homolog genes was determined to be the indicator for germ cell differentiation. In embryoid bodies that are generated via float culture of ES cells, differentiation occurs in various cell species including triploblasts as in the case of differentiation in the organisms, and expression frequency of germ cells is considered to be very low. Accordingly, a differentiation of interest cannot be accurately identified via conventional molecular genetic detection or immunohistochemical staining. In the present invention, a recombinant expression vector was constructed as a means for overcoming such problem. Such recombinant expression vector is constructed by performing genetic engineering whereby the expression of Vasa homolog genes is replaced with the expression of marker genes such as GFP (i.e., a green fluorescent protein of luminescent jellyfish) or lacZ (i.e., bacteria-derived galactosidase enzyme; capable of blue color development or fluorescence emission in accordance with a chromogenic substrate). Such expression vector comprises the marker genes under the control of the expression of Vasa homolog gene.

Use of a foreign gene expression vector utilizing an expression control region of a Vasa homolog gene is particularly effective in gene function analysis and tissue engineering applications involving germ cell lineages. Marker genes used in the present invention are not particularly limited, provided that the expression of such genes can be easily detected or assayed. Examples thereof include genes which encode fluorescent proteins such as GFP (green fluorescent protein), RFP (red fluorescent protein), BFP (blue fluorescent protein), and YFP (yellow fluorescent protein), chemiluminescent enzyme genes such as lacZ, and drug-resistant genes such as a neomycin-resistant gene. Artificially synthesized genes, which are prepared by adding various mutations to the aforementioned genes, can also be used.

In the present invention, the term "mammalian-derived Vasa homolog genes" refers to Vasa genes of mammalian animals, such as a mouse, human, pig, calf, sheep, or rat. These Vasa homolog genes are known. For example, mouse Vasa genes are described in Fujiwara, Y. et al., Proc. Natl. Acad. Sci. U.S.A., 91, 12258-12262, 1994; human Vasa genes are described in Castrillon, D. H. et al., Proc. Natl. Acad. Sci. U.S.A., 97, 9585-9590, 2000; and rat Vasa genes are described in Komiya et al., Dev. Biol., 162, 354-363; 1994.

Hereafter, specific explanation is provided with reference to a case in which the mammalian-derived Vasa homolog genes are mouse Vasa homolog genes.

The expression control region of the Mvh gene was identified by the following two methods.

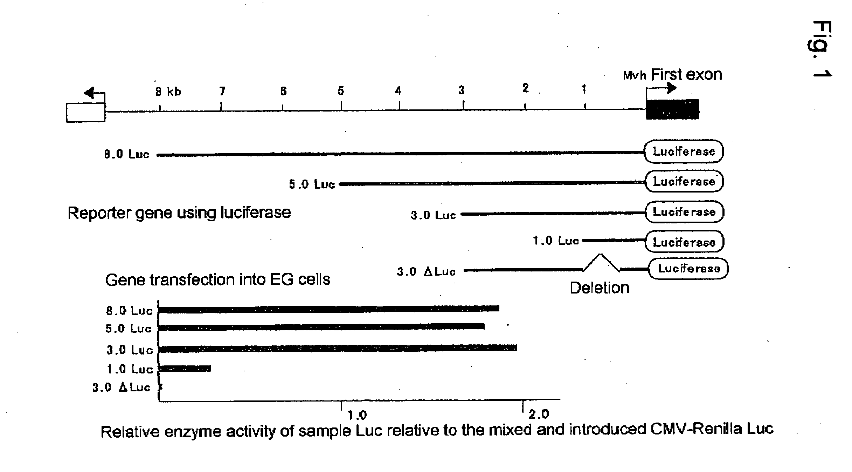

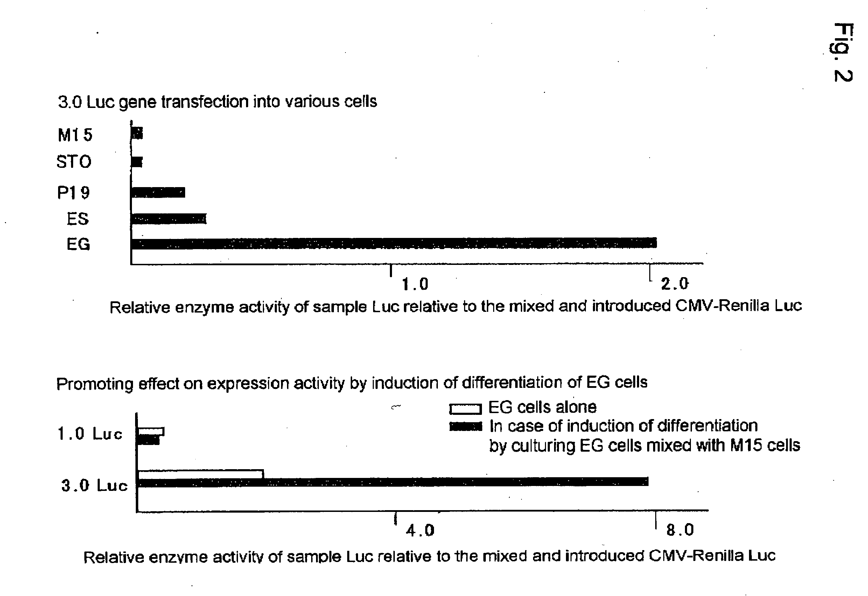

The first method is a method for assaying promoter activity wherein genomic DNA upstream of the first exon is isolated in various lengths and assay is carried out using a firefly luciferase enzyme protein or the like as a reporter gene. In the present invention, the term promoter activity" refers to transcription activity of a basic promoter and transcription-accelerating activity of an enhancer, and an expression control sequence having such promoter activity is referred to as a "promoter sequence." Specifically, the length of a DNA sequence located upstream of the transcription initiation point including a GC-rich basic promoter is adjusted to a given length, the sequence is ligated to a luciferase-containing promoter vector, the genes are introduced into EG (embryonic germ) cells, which are cultured germ cells, via lipofection, and expression activity is detected via the luciferase enzyme activity assay method. As a result, potent transcription-accelerating activity was observed in the region from the transcription initiation point up to about 3.0 kb upstream thereof, as shown in Fig. 1. Hereafter, the term "upstream" refers to a "region upstream of the transcription initiation point," unless otherwise specified. Weak activity observed at a region 1.0 kb upstream is considered to indicate the activity of a basic promoter. This is also verified by the fact that activation of a region 1.0 kb upstream is not influenced by cell species and that deletion of such region results in complete loss of transcription activity, as shown in Fig. 2. (Fig. 1). Transcription-accelerating activity at a region 3.0 kb upstream has cell specificity, and the expression level is enhanced when EG cells are mixed and cultured with gonadal somatic cells (M15). Such phenomena are consistent with the behavior of endogenous Vasa expression. This demonstrates that a region 1.0 kb to 3.0 kb upstream at least comprised a major germ cell-specific expression regulating sequence.

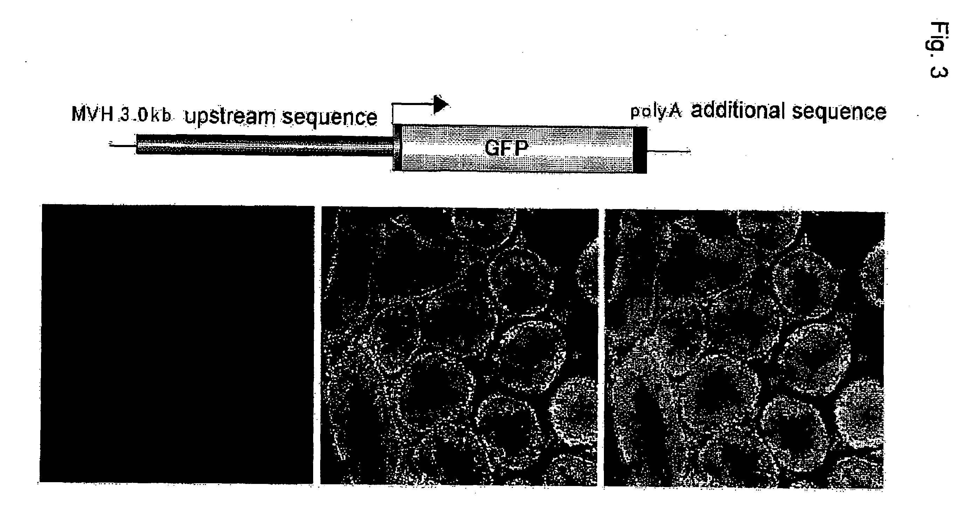

According to the second method, the Vasa expression control region that was determined in the above cell culture system was examined using a transgenic (Tg) mouse obtained by microinjecting a foreign gene into a fertilized mouse egg. For example, GFP expression specific for intratesticular germ cells is detected in a transgenic mouse of the 3.0-GFP gene comprising a GFP gene, as a reporter, ligated to a region 3,0 kb upstream of the transcription site. Such GFP expression is identified as a cell that emits green fluorescence under a fluorescent microscope, and the most positive expression is observed in spermatocytes in the meiotic phase to post-meiotic spermatids (Fig. 3). The same results were observed when a wider range, i.e., the region 8.0 kb or 5.0 kb upstream, was employed.

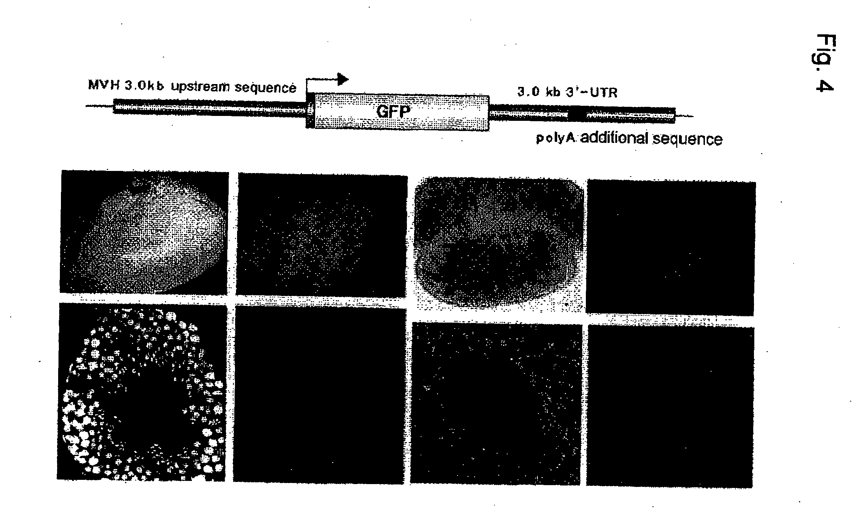

With the use of such transgenic mouse comprising an upstream region only, however, expression levels of fetal primordial germ cells or female germ cells such as oocytes of Mvh are less than the detection sensitivity, and it is suggested that a control sequence that plays a role in enhancing the expression level is present in regions other than the upstream region. For example, the 3'-untranslated region of the Mvh gene is deduced to be capable of regulating translation activation that is in charge of a long-term protein expression in germ cells *in vivo.* In fact, with the use of a transgenic mouse comprising a 3.0-GFP-UTR gene prepared by ligating a 2.3-kb region located downstream of the translation stop codon at the end of the Mvh translation region to a site downstream of the 3.0-GFP gene, weak GFP expression can be visualized in fetal primordial germ cells, and significant expression can be observed via GFP antibody staining (Fig. 4).

Thus, at least two control elements, i.e., specific transcription-accelerating activity via a region upstream (about 3.0 kb) of the transcription site and translation activation via a 3'-downstream region containing an untranslated region, were demonstrated to be effective in order to regulate Mvh gene expression.

A nucleotide sequence comprising a region from a site 3 kb upstream of the transcription initiation point of the Mvh gene to the transcription initiation point, that can be used for the recombinant expression vector of the present invention, is shown in SEQ ID NO: 1 of the Sequence Listing. A nucleotide sequence of the 3'-untranslated region (UTR) sequence of the Mvh gene that can be used for the recombinant expression vector of the present invention is shown in SEQ ID NO: 2 of the Sequence Listing. Promoter sequences of mammalian-derived Vasa homolog genes that can be used for the recombinant expression vector of the present invention are not limited in terms of original animal species. It is preferable to use an animal species that is the same as the animal into which the expression vector is to be introduced or the animal from which the cell to be introduced is derived. Also, it is preferable that such genes be expressed in a manner specific to the differentiated germ cells.

The recombinant expression vector of the present invention can adequately comprise elements that are used for general expression vectors, such as selection marker genes such as ampicillin resistant genes and a replication origin, in addition to the aforementioned elements.

### (2) Cells and transgenic non-human mammalian animals into which the recombinant expression vectors of the present invention have been introduced

The present invention provides cells that are obtained by introducing the recombinant expression vector according to (1) above. Examples of cells into which the recombinant expression vectors of the present invention are to be introduced include ES cells and tissue stem cells. Examples of tissue stem cells that can be used include mesenchymal cells, and particularly, bone marrow mesenchymal cells. ES cells and tissue stem cells derived from mammalians or avians can be used. Use of mammalian-derived cells is preferable, and use of mouse- or human-derived cells is particularly preferable. Specific examples of ES cell lines that can be used in the present invention include the E14 cell strain derived from a male mouse 129 strain, the E14TG2a cell strain derived therefrom, and the human ES cell strain H9 (Thomson et al., Science, 282, 1145-1147, 1998). The recombinant expression vector of the present invention may be introduced into such cells to separate germ cells and differentiated cells.

A recombinant expression vector can be introduced into a cell by a method known in the art. Specific examples of methods that can be used include electroporation, the calcium phosphate method, and lipofection. Also, a method involving the use of a commercially available transfection reagent such as Lipofectamine 2000 (Invitrogen) can be employed.

Further, the present invention provides a transgenic non-human mammalian animal into which the recombinant expression vector of the present invention has been introduced.

A method for producing the transgenic non-human mammalian animal of the present invention is not particularly limited. For example, the transgenic non-human mammalian animal of the present invention can be produced by introducing the recombinant expression vector of the present invention into a fertilized egg. Specifically, the recombinant expression vector of the present invention is introduced into a fertilized egg of a non-human mammalian animal, the fertilized egg is transplanted into a pseudopregnant female non-human mammalian animal, and such non-human mammalian animal is made to deliver a non-human mammalian animal into which such recombinant expression vector has been introduced. Thus, the transgenic non-human mammalian animal of the present invention can be produced.

Examples of non-human mammalian animals that can be used include rodents (such as mice, hamsters, guinea pigs, rats and rabbits), dogs, cats, goats, sheep, cows, pigs, and monkeys. Rodents such as mice, hamsters, guinea pigs, rats, and rabbits are preferable from the viewpoint of ease of preparation, growth, and use, with mice are being the most preferable.

At the fertilized egg stage, the recombinant expression vector of the present invention can be introduced so as to be present in all germline and somatic cells of the target mammalian animal. If the recombinant expression vector of the present invention is present in the germline cells of the animal produced after gene introduction, this indicates that the recombinant expression vector of the present invention is present in all the germline and somatic cells of all the progenies. The offspring of this animal species that has inherited the gene of interest comprise in their germline and somatic cells the recombinant expression vector of the present invention.

The transgenic animal of the present invention can be continuously raised under general conditions as an animal containing the gene of interest, after such animal is confirmed to stably maintain the gene via crossing. Homozygote animals containing introduced genes in both homologous chromosomes are obtained, and breeding and subculture can be performed in such a manner that all the offspring have the genes of interest by crossing males and females of such homozygote animals.

Hereafter, a specific explanation is provided with reference to a case in which a transgenic non-human mammalian animal is a transgenic mouse. The recombinant expression vector of the present invention is microinjected into a male pronucleus of a fertilized mouse egg, the resulting egg is cultured, the cultured egg is transferred to an oviduct of a pseudopregnant female mouse, the transplanted animal is grown, and an offspring mouse having the aforementioned expression vector is selected from among the born offspring mice. Thus, a transgenic mouse of the present invention can be produced. As the fertilized mouse egg, any egg obtained by crossing mice derived from, for example, 129/sv, C57BL/6, BALB/c, C3H, or SJL/Wt can be used.

An offspring mouse comprising the recombinant expression vector of the present invention can be selected by, for example, dot-hybridization wherein DNA is extracted from a murine tail or the like and the introduced recombinant expression vector is used as a probe, or PCR involving the use of specific primers.

### (3) Method for obtaining germ cells

The present invention further provides a method for obtaining germ cells which comprises recovering cells capable of differentiating germ cells from a transgenic non-human mammalian animal into which the recombinant expression vector of the present invention has been introduced by employing expression of said marker gene as an indicator. The present invention also provides a method for obtaining germ cells which comprises culturing an ES cell or tissue stem cell into which the recombinant expression vector of the present invention has been introduced and recovering cells capable of differentiating germ cells by employing expression of the marker genes as an indicator.

As germ cells to be obtained, germ stem cells are preferable. The term "germ stem cells" used herein may refer to either sperm stem cells or egg stem cells.

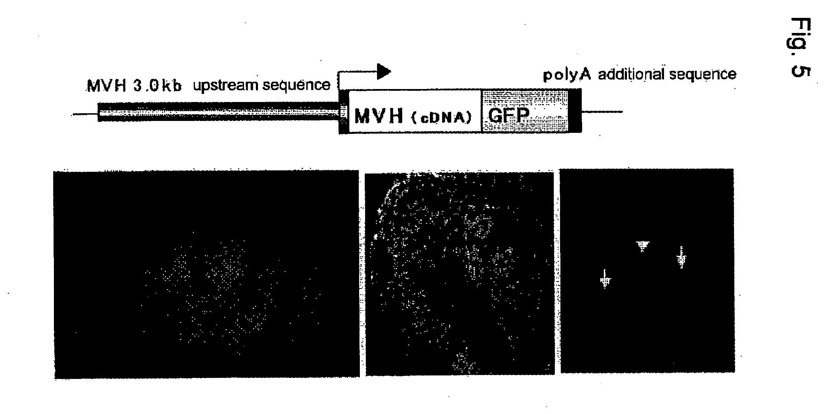

When a foreign gene is to be expressed using an upstream 3.0-kb region of the Mvh gene as a control promoter sequence, such expression can be limited to cells after the meiotic stage of spermatogenic cells. Such specificity can be utilized for function analysis of gene products specific to the spermatogenic process or monitoring of intracellular behavior of a foreign gene. A concrete example involves the intracellular locality of a Vasa-GFP-fused protein. By the antibody staining method, the MVH protein is found to be localized in a uniformly dispersed state in the cytoplasm during the mouse spermatogenic process, granulated during the meiotic stage, and localized as a single giant granule (chromatoid body (CB)) adjacent to a nuclear membrane in the sperm cell (Toyooka, Y. et al, Mech. Dev., 93, 139-149, 2000). The CB construct is a composite of a protein and an RNA component and is an intracellular organ that is deduced to function as a translation regulator at the time of sperm generation. Germ cell-specific expression of Vasa-GFP fusion protein enables observation of such CB construct under nonfixed conditions. Expression levels in 3.0-kb upstream regions are as low as about one fifth of the expression levels of endogenous Mvh genes, and thus they do not inhibit physiological functions such as sperm generation or influence fertility. As shown in Fig. 5, behavior of MVH-GFP which is a foreign gene is consistent with the behavior of endogenous Mvh protein and localized in cytoplasmic granules in spermatocytes and in the CB construct in sperm cells. Such characteristics enable real-time assay of behavior of the CB construct over time. Further, they also provide a novel means for purifying factors that are associated with generation or functions thereof from unfixed samples. A similar method can be applied to protein factors that are involved in sperm formation and can be utilized for visual labeling and identification of coupling factors.

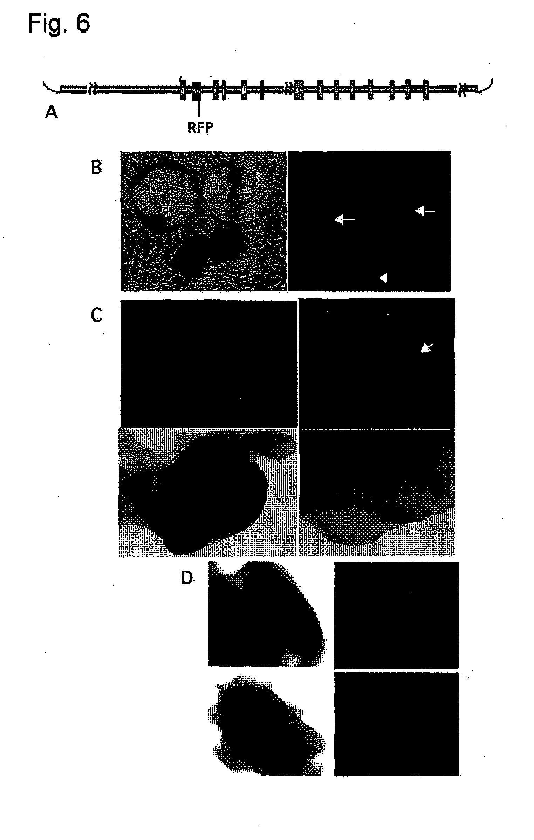

As a method for constructing a foreign vector that accurately reproduces expression specificity of the endogenous Mvh gene, giant DNA containing the full-length Mvh (or Vasa homolog genes of various animal species) genomic gene can be cloned into a BAC vector or the like (cosmid, P1 phage), and a foreign gene expressed in the resulting vector can be incorporated into an Mvh gene. In such a case, the full-length Mvh gene is a sequence containing at least an about-60-kb region from a site about 3.0 kb upstream of the transcription initiation site to a site about 3.0 kb downstream of the translation termination site, and cDNA of a foreign gene is effectively incorporated in such a manner that a translation codon is in frame with a codon downstream of the translation initiation site, or a gene comprising a ribosome-binding sequence (ires) ligated to a site upstream of a foreign gene can be effectively inserted or substituted. A specific example is an example concerning a reporter gene comprising an RFP gene incorporated into a BAC clone having about 150-kb genomic DNA containing the Mvh gene. As shown in Fig. 6A, a Bac-Vas-RFP vector into which cDNA of the RFP gene is inserted so as to be positioned with the translation initiation site of the second exon is used for gene introduction into cultured cells via lipofection or for preparation of a transgenic mouse via DNA microinjection. In the case of gene introduction involving the use of ES cells as host cells, Bac-Vas-RFP recombinant ES cells can be selected in accordance with drug resistance to be incorporated (i.e., selection in accordance with G418 resistance of a Neo-resistant gene in this case). Recent studies demonstrated that cell species including primordial germ cells, spermatogenic cells, and oocytes are differentiated during culture from ES cells (Toyooka, Y. et al, Proc. Natl. Acad. Sci. U.S.A., 100, 11457-11462,2003; Hubner, K. et al, Science, 300, 1251-1256 2003; Geijsen, N. et al., Nature, 427, 147-154, 2004). Accordingly, ES cells into which the recombinant expression vector of the present invention has been introduced enable identification of Mvh-expression-positive germ cells as RFP red fluorescent cells in accordance with differentiation induction during culture. Also, germ cells that have been differentiated into different developmental phases via culture can be selectively purified using a cell sorter (Fig. 6B). In the case of the transgenic mouse lineage (BR-15) into which a Bac-Vas-RFP vector has been incorporated, germ cells in the testis and the ovary thereof, i.e., spermatogenic cells at every stage from the spermatogonium stage to the sperm stage and oogenic cells at every stage from the oogonia stage to the mature egg stage are identified as red fluorescent cells resulting from RFP expression (Fig. 6C). Such significant reporter expression is detected at earlier stage than the case involving the use of an upstream 3,0-kb promoter sequence, such expression is consistent with expression of endogenous Mvh, and the genes of interest can be detected from primordial germ cells of male and female fetal reproductive glands (Fig. 6D).

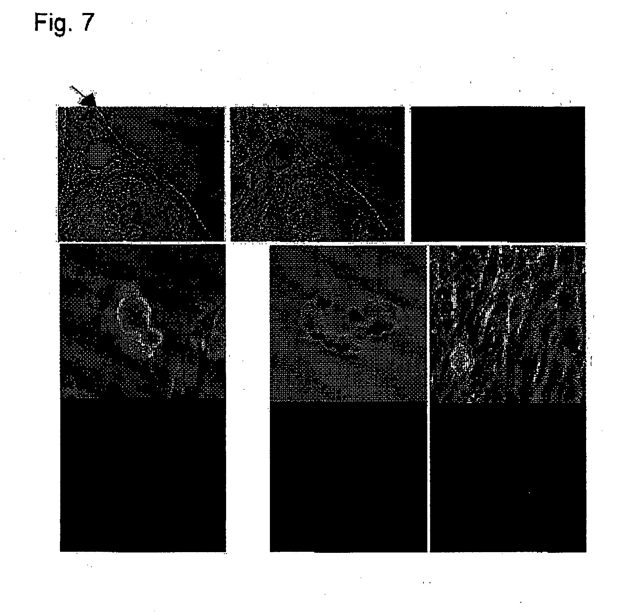

It is also known that an adult testis comprises sperm stem cells that are in charge of periodical spermatogenesis and that have properties different from those of primordial germ cells or gonocytes, which are undifferentiated germ cells. Also, a method for culturing the same has been established in recent years, sperm or offspring derived from cultured sperm stem cells can be established via transplantation into testis of living organisms, and sperm stem cells draw attention as an excellent new material in the field of reproductive engineering (Kanatsu-shinohara, M., et al, Biol. Reprod., 69, 612-616, 2003). The aforementioned Bac-Vas-RFP can visualize and identify such sperm stem cells. This was verified in the following manner. That is, when adult murine sperm cells were cultured under the conditions for culturing sperm stem cells, grown sperm stem cell clones were specifically identified as RFP-positive cells (Fig. 7A). Separately, the presence of oocyte stem cells that function as stem cells for oogenesis in the adult ovary was reported in recent years. Such cell species are identified as Mvh expression-positive cells located in the basal membrane surrounding the entire ovary or outside thereof (Johnson, J. et al, Nature, 428, 145-150, 2004). While oocytes in the ovary have a large spherical shape with a cytoplasm, the oocyte stem cells have the flat shape according to a cross-sectional image of tissue. In tissue samples of the Bac-Vas-RFP mouse ovary, oocyte stem cells located outside the basal membrane are also observed as RFP-positive cells, as with the case of oocytes (Fig. 7B). As shown in Fig. 7C, when dispersed ovary cells are cultured, none of the oocytes at the meiotic stage grow; however, RFP positive cells that are capable of proliferation or cells that maintain a flat shape are also observed. This indicates that labeling of cells with Bac-Vas-RFP vectors enables identification and purification of oocyte stem cells.

With the use of properties of the recombinant expression vector of the present invention that involves the use of the expression control regions of the mammalian Vasa homolog genes as described above, germ cells can be identified from biological tissues of transgenic animals prepared with the use of such recombinant expression vector. This is particularly effective for specifically selecting and culturing germ stem cells, and foreign gene expression enables fluorescent labeling or addition of foreign traits such as drug selectivity or novel physiological factors. Such specificity can also be utilized in a cell culture system. Besides mice, genes may be introduced into cultured cells derived from various biological tissues such as ES cells or tissue stem cells of other animal species, including humans. Thus, cells that are induced to differentiate into or differentiated and converted into germ cell lineage by culture can be isolated. In particular, pure culture of germ stem cells can be effectively established from separate culture of cells differentiated into germ stem cells.

### (4) Method for screening for germ cell differentiation enhancing factor

The present invention further provides a method for screening for a germ cell differentiation enhancing factor which comprises administering a test substance to a transgenic non-human mammalian animal into which the recombinant expression vector of the present invention has been introduced and selecting a test substance that induces expression of the marker gene, and a method for screening for a germ cell differentiation enhancing factor which comprises culturing an ES cell or tissue stem cell into which the recombinant expression vector of the present invention has been introduced in the presence of a test substance and selecting a test substance that induces expression of the marker gene. The germ cell differentiation enhancing factors obtained by such screening methods are within the scope of the present invention.

Types of test substance are not particularly limited. Examples thereof include peptide, polypeptide, a synthetic compound (e.g. a low-molecular-weight organic compound and a high-molecular-weight organic compound), a fermented microorganism, an extract from an organism (including, for example, plant or animal tissue, microorganisms, and cells), and a library of any thereof. Examples of libraries include a synthetic compound library (e.g., a combinatorial library) and a peptide library (e,g., a combinatorial library). A test substance to be screened for may be naturally occurring or synthetic, a single candidate test substance may be independently tested, or a mixture of several candidate test substances (including libraries) may be tested. Also, a mixture such as a cell extract may be fractionated, the resulting fractions may be subjected to screening, and fractionation may be repeated to isolate a substance that induces expression of the marker gene in the end.

### (6) Method for testing toxicity to germ cells

The present invention further provides a method for testing toxicity to germ cells which comprises administering a test substance to a transgenic non-human mammalian animal into which the recombinant expression vector of the present invention has been introduced and detecting a substance having toxicity to germ cells by employing expression of such marker gene as an indicator, and a method for testing toxicity to germ cells which comprises culturing an ES cell or tissue stem cell into which the recombinant expression vector of the present invention has been introduced in the presence of a test substance and detecting a substance having toxicity to germ cells by employing the number of cells in which expression of the marker gene is induced as an indicator.

A type of a substance (a test substance) that is toxic to germ cells is not particularly limited. Examples thereof include peptide, polypeptide, a synthetic compound (e.g. a low-molecular-weight organic compound and a high-molecular-weight organic compound), a fermented microorganism, an extract from an organism (including, for example, plant or animal tissue, microorganisms, and cells), and a library of any thereof. Examples of libraries include a synthetic compound library (e.g., a combinatorial library) and a peptide library (e.g., a combinatorial library). A test substance may be naturally occurring or synthetic, a single candidate test substance may be independently tested, or a mixture of several candidate test substances (including libraries) may be tested. Also, a mixture such as a cell extract may be fractionated, the resulting fractions may be subjected to screening, and fractionation may be repeated to isolate a substance that induces expression of the marker gene in the end. More specifically, an endocrine disrupter (i.e., an environmental hormone) or the like may be evaluated as a substance that is toxic to germ cells by the method for testing toxicity of the present invention.

Hereafter, the present invention is described in greater detail with reference to the following examples, although the technical scope of the present invention is not limited thereto.

### Examples

### Example 1: Assay of transcription control region of Mvh gene

The Mvh genomic gene region was prepared by isolating a clone containing the first exon of the Mvh genomic gene from the 129 mouse genome library (EMBL3, Clontech) based on the restriction map of the insert DNA thereof. The regions 5.0 kb, 3.0 kb, and 1.0 kb upstream are fragments comprising a region from the SmaI site in the first exon to the KpnI site about 5.0 kb upstream thereof, a region therefrom to the PstII site about 3.0 kb upstream thereof, and a region therefrom to the EcoRV site about 1.0 kb upstream thereof. These fragments were inserted into the cloning sites of the Basic-Luc vector (pGL3-basic, Promega) in the same 5'-3' direction to prepare 5.0 Luc, 3.0 Luc, and 1.0 Luc assay vectors. The 8.0 Luc vector was prepared from the 1.0 Luc vector by inserting an about 7.8-kb NotI genome fragment located immediately before the first exon of the genome. The 3.0Δluc vector lacks a XbaI/SacI fragment (corresponding to a region -400 to -900 kb upstream) of the 3.0 Luc vector (see Fig. 1).

EG cells as host cells for gene introduction are cultured cells established from primordial germ cells of the mouse embryo 8.5 days after fertilization, and such cells are grown in DMEM medium containing 10% FCS (fetal calf serum), bFGF (100 ng/ml), and LIF (10,000 units/ml). Cells that had been cultured at a density of about 1,000,000 cells per well on a 12-well plate were employed as an assay sample. In the case of gene introduction involving the use of 2 µg of an analyte, Lipofectamine 2000 (Invitrogen) was used, and the method of introduction was in accordance with the manufacturer's instructions. CMV-Renilla (sea pansy) Luc vector (pRL-CMV, Promega) was used as an internal control for standardizing the introduction efficiency; and the analyte and the control were simultaneously introduced at the ratio of 100:1 per assay. Cell samples were recovered 1 day after the introduction, and enzyme activity was assayed using the dual luciferase assay system (Promega). Fig. 1 is a chart indicating relative activity ratio in relation to the internal control, CMV-Renilla Luc.

Fig. 2 shows the results of assay wherein the 3.0 Luc vector exhibiting significant expression accelerating activity was assayed using various mouse cell lines as host cells. The cell lines used were the aforementioned EG cells (Matsui, Y. et al., Cell, 70, 841-847, 1992), and M15 (the supporting cell lineage in the fetal gonad of a 10.5-day-old mouse embryo) (Larsson, S. H. et al., Cell, 81,391-401,1995), P19 (an embryonic carcinoma cell) (McBurney, M. W. & Rogers, B. J., Dev. Biol., 89, 503-508, 1982), STO (fibroblasts) (Martin, G., Proc, Natl. Acad. Sci. U.S.A., 78, 7634-7639, 1981), and ES (CCE, an embryonic stem cell line) (Robertson, E. et al., Nature, 323, 445-449, 1986).

Assay was carried out in the same manner as shown in Fig. 1. As a result, the 3.0 Luc vector was found to have expression accelerating activity specific to EG cells derived from germ cells. As shown in Fig. 2, some expression is observed in ES cells; however, significant expression is observed in EG cells. This indicates that there is no expression in ES cells, or very low expression. Such result does not indicate that introduction into ES cells is invalid, but it supports the assumption that reproduction and differentiation following introduction into ES cells can activate expression. Also, the expression level of the endogenous Mvh genes is significantly enhanced after germ cells are fixed on the reproductive glands, and it is also observed when EG cells are mixed and cultured with the gonad supporting cell lines. Whether or not such accelerating effects were reproduced in the case of the 3.0 Luc vector was examined. As a control, EG cells alone and a mixed (1:1) culture of EG cells and M15 cells were used as hosts to perform the same assay as described above with the use of the 1.0 Luc vector. As a result, the 3.0 Luc vector was found to exhibit significant acceleration effects. These results indicate the presence of an element sequence that is in charge of Mvh expression-specificity in a region 1.0 to 3,0 kb upstream.

### Example 2: Production of 3.0-GFP transgenic (Tg) mouse and analysis of properties thereof

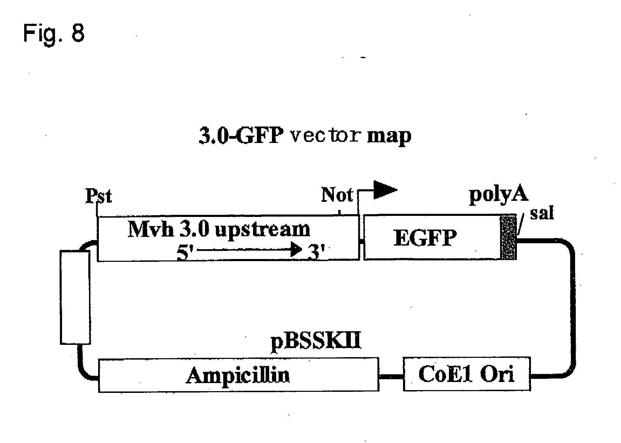

The fragment lacking the luciferase gene portion of the 3.0 Luc vector prepared in Example 1 was ligated to the EcoRI-SalI fragment of the EGFP gene which was isolated from the pEGFP-promoter vector (Clontech) so as to prepare a 3.0-GFP vector. The structure of the 3.0-GFP vector is shown in Fig. 8. Such DNA was cleaved at the ScaI site in the vector, and linearized DNA solution (10 ng/µl) was microinjected into the pronucleus of the fertilized mouse (B6C3F1) egg to establish the Tg mouse 3 lineages into which the 3.0-GFP vector has been introduced.

Specifically, the fertilized egg into which DNA of the expression vector had been microinjected was transplanted to the oviduct of the pseudopregnant mouse, and a genetically recombinant mouse was delivered from the false parent mouse. Thus, a transgenic mouse of interest could be produced. Whether or not the offspring mouse was a recombinant was determined by cleaving the chip of the tail of the mouse, extracting genomic DNA from the tissue thereof, treating the DNA with EcoRI restriction enzyme, developing the product via agarose electrophoresis, and analyzing the product via Southern blotting using a probe prepared by labeling the GFP fragment with 32P-dCTP. The resulting offspring recombinant mice were independently subjected to crossing and breeding to establish mouse lineages comprising expression vectors incorporated into different chromosome sites.

The thus-produced Tg mouse 3 lineages comprising the 3.0-GFP vector incorporated therein exhibited the identical results. The adult testis of the resulting Tg mouse also exhibits a GFP-derived green fluorescence at the organ level under a fluorescent stereoscopic microscope. Fig. 3 shows the cross-sectional image of the tissue of the Tg mouse testis. The testis was immobilized with a 4% paraformaldehyde solution and then frozen-sectioned. A TO-PRO3 iodide solution (Molecular Probes) was used for nuclear staining in order to visualize tissues. As a result, GFP fluorescence was detected from spermatocytes in spermatogenic cells to a spermatid layer in the seminiferous tubule. Similar detection was performed concerning the fetal reproductive glands and ovaries; however, expression thereof was not detected in the 3.0-GFP Tg mouse. Accordingly, the Mvh upstream promoter region was found to be expressed in male germ cells after the meiotic period *in vivo.*

### Example 3: Production of 3.0-GFP-UTR Tg mouse and analysis of properties thereof

In the upstream 3.0-kb region, expression at the fetal stage or in the ovary was not reproduced. This indicates the presence of translation-regulating activity of an untranslated transcription region (3'-UTR) located downstream of the translation region. Thus, a 3.0-GFP-UTR Tg mouse comprising 3'-UTR of the Mvh gene ligated downstream of the 3.0-GFP of Example 2 was produced. The 3'-UTR fragment used was isolated by PCR involving the use of the Mvh genomic clone (the entire genomic DNA of a murine cell may be used) as a template. Primers used here were 5'-GATTAAAGCAAACGAACATA-3' (SEQ ID NO: 3) and 5'-CCCACTGCACATAGGACTTAT 3' (SEQ ID NO: 4), and the resulting 2.3-kb product comprises the entire about-600-b region from the translation stop codon to the untranslated transcription region. PCR was carried out using Ex-taq or LA-taq (Takara Shuzo) by repeating a cycle of 94°C for 1 minute, 58°C for 2 minutes, and 72°C for 3 minutes 27 to 30 times. Such PCR conditions are employed in all examples unless otherwise specified. Thus, description of PCR conditions is omitted in the subsequent examples. The PCR products were subcloned into the pGEM-T Easy vector (Promega) and cleaved using NotI sites located at both ends of the cloning site, the cleaved fragment was inserted into the NotI site located downstream of GFP of the 3.0-GFP vector in the same orientation, and the resultant was designated as the 3.0-GFP-UTR vector. Tg mice were produced in the same manner as in Example 2.

Fig. 4 shows the results of in vivo GFP fluorescent observation and tissue section observation of the adult testis and the fetal testis of the resulting 3.0-GFP-UTR Tg mouse. While GFP fluorescent expression at the fetal stage was weaker than that in the adult testis, such expression was significant. With the use of such tissue section, the detection could be carried out by the fluorescent antibody method using an anti-GFP antibody (1/500-fold, Clontech) in combination with the Oregon Green-anti-rabbit IgG (1/500-fold, Molecular Probes). Based on such results, it is concluded that use of a 3.0-kb upstream region in combination with an up to 2.3-kb downstream region is effective when expression specificity of Mvh genes is used.

### Example 4: Production of 3.0-VASA-GFP Tg mouse and analysis of visualized protein behavior

The results of Example 2 indicate that Mvh 3.0 kb exhibits activity of inducing specific expression of genes ligated downstream in spermatogenic cells in the testis. For an example of analyzing behavior of the visualized protein molecules with the utilization of such specificity, a 3.0-VASA-GFP Tg mouse was produced. This was intended to ligate fusion cDNA of VASA-GFP to a site downstream of Mvh 3.0 kb so as to visualize variations in intracellular distribution of VASA proteins by means of GFP fluorescence. A vector was constructed based on modification of 3.0-GFP vector. At the outset, a plasmid clone containing the full-length Mvh cDNA (or cDNA prepared from the testis mRNA) was used as a template to prepare cDNA having the BamHI site at the 5' end and the KpnI site at the 3' end of the full-length Mvh translation region via PCR. Primers used were 5'-BamHI-GAAGCTATCATGGGAGATGAA-3' (SEQ ID NO: 5) and 5'-KpnI-GGCCCATGATTCGTCATCAA-3' (SEQ ID NO: 6). Similarly, GFP cDNA having the KpnI site at the 5' end and the SalI site at the 3' end was prepared via PCR. Primers used were S'-KpnI-ACCATGGTGAGCAAGGGCGAG-3' (SEQ ID NO: 7) and 5'-SalII-CTACTCAGACAA TGCGATGC -3' (SEQ ID NO: 8). The resulting two PCR fragments were subcloned into the pGEM-T Easy plasmid, and a fragment cleaved with KpnI/Sall from another GFP plasmid was ligated to the Mvh plasmid that had been ring-opened with KpnI/Sall. Thus, Mvh and GFP were ligated to each other while sandwiching two codons at the KpnI site so as to construct a single fusion cDNA. The Mvh-GFP cDNA was cleaved with BamHI/SalI, and the resulting fragment was inserted into a ring-open vector prepared by cleaving GFP cDNA via BglII/SalI cleavage from the 3.0-GFP vector so as to construct a 3,0-VASA-GFP vector.

A method for producing Tg mice and a method for observing a fluorescent image of the testis tissue of such mice are the same as those employed in the above examples. As shown in Fig. 5, in such Tg mouse testis, expression of VASA-GFP fusion proteins is observed in oocyte cells and testicular cells. Visualization of the chromatoid body and observation of dynamics in viable cells were possible in order to examine the peculiar intracellular locality.

### Example 5: Visualization of germ cells using BAC-Vasa-RFP gene

Tg genes were prepared using BAC vector basically in accordance with the method developed by Lee et al. (Lee E-C et al. Genomics 73, 56-65, 2001). BAC-cloned E. coli comprising an about 140-kb mouse genomic DNA containing the Mvh locus as an insert was isolated from the mouse genomic BAC library (Clontech). A DNA fragment that induces homologous recombination in the BAC clone is prepared via PCR using Pfu polymerase (Promega). When RFP gene introduction is intended, plasmid DNA comprising a RFP cDNA (Clontech) fragment subcloned into pUC-loxP-Neo-loxP in advance is used as a template. The 5' and 3' primers used are a 5'-50-mer region [homologous sequence A]-TGTGGAATTGTG,A,GCGGATA-3' (SEQ ID NO: 9) and a 5'-50-mer region [homologous sequence B]-GTTTTCCCAGTCACGACGTT -3' (SEQ ID NO: 10). The homologous sequence A is a 50-nucleotide-long sequence comprising a region up to the translation initiation point ATG of Mvh located in the Mvh second exon, and the homologous sequence B is a 50-nucleotide-long complementary sequence comprising a region thereafter. The resulting PCR product was mixed with recombinant-competent BAC-cloned E. coli, the resultant was introduced, culture was conducted on a chloramphenicol/kanamycin plate, and clones containing homologous recombinant BAC were isolated from drug-resistant E. coli. BAC DNA was prepared using a Nucleobond-ax column (Macherey-Nagel) and used for preparation of Tg mice and introduction into ES cells (Fig. 6A).

BAC DNA was introduced into a wild-type ES cell (E14) using Lipofectamine 2000, and the recombinant clone was selected based on G418 drug resistance. Embryoid bodies were prepared by transferring ES cells (1,000,000 cells/ml) to DMEM medium containing 10% FCS and using a suspension culture plate (Nunc). ES cells form cell aggregates and undergo triploblastic differentiation during culture therein. In the case of a BAC-Vasa-RFP recombinant, RFP red fluorescence-positive cells were clearly observed about 3 days later, and germ cells differentiated during culture were identified (Fig. 6B). In the case of BAC-Vasa-RFP Tg mice, primordial germ cells in the testis, ovary, and fetal gonad were detected as clear RFP red fluorescence-positive cells (Fig. 6C). Visualization of oocytes in the ovary became possible for the first time with the use of such Tg mice. A reason therefor was the prolonged life of a reporter RFP protein. Such significant reporter expression was detected earlier than the case in which the upstream 3.0-kb promoter was used, and such reporter expression can be detected from the primordial germ cells of male and female fetal gonads as with the expression of endogenous Mvh (Fig. 6D).

### Example 6: Identification and culture of germ stem cells using BAC-Vasa-RFP

With the use of the Tg mice produced in Example 5, identification of stem cells, which were difficult or impossible to visualize, was found to be possible. As shown in Fig. 7, Mvh-positive cells located on a basal membrane of the ovary or outside the tissue thereof can be identified as red fluorescent cells in the form of biological tissue in this mouse lineage. Such advantage enables monitoring of the morphology or growth conditions of the putative oocyte stem cells without losing sight thereof during the process of primary tissue culture. As an example, culture of the ovary of a 10-day-old offspring BAC-Vasa-RFP mouse is shown. The ovary extracted from the organism is subjected to dispersion treatment for a short period of 5 minutes in a 0.25% trypsin solution (Gibco) to recover soft tissue portions in the ovary as dissociated cells. The resultant was suspended in 10% FCS-containing DMEM medium and cultured on a gelatin-coated culture plate overnight. Thereafter, the medium is repetitively substituted with STEM medium that had been developed for sperm stem cells (Kanatsu-shinobara, M., et al, Biol. Reprod., 69, 612-616, 2003), and long-term culture is then conducted. During such process, a giant cell mass, i.e., an ovarian follicle, and giant spherical cells, i.e., oocytes, are suspended and they are thus removed via culture medium exchange. About 1 week thereafter, RFP red fluorescence, i.e., indicating Mvh positive germ cells, was found to be dispersed in a cell layer adhered to the plate bottom surface (Fig. 7C). These cells are classified into cells exhibiting colony morphology resulting from mitosis, and unicellular and spindle-shaped cells. These cells are apparently different from oocytes in terms of size, morphology and division potential, and are considered to derive from oocyte stem cells observed in biological tissues. Such cell species can be identified during culture. This indicates the possibility of establishing clone cell lines via fluorescence-based cell selection. Also, sperm stem cells, establishment of cultured cells of which had become already possible, were found to be possible to be identified, and establishment of red-fluorescent sperm stem cell lines via culture using the testis-derived cells of the BAC-Vasa-RFP mouse was also possible (Fig. 7B). The method for culturing sperm stem cells was in accordance with the method reported by Kanatsu-Shinohara et al. (Biol. Reprod., 69, 612-616, 2003).

### Industrial Applicability

According to the present invention, a transgenic non-human mammalian animal and a cultured cell system were established, and they enable highly sensitive detection of expression of Vasa homolog genes that are not expressed in undifferentiated stem cells but are expressed specifically in differentiated germ cells. The expression vector of the present invention provides an excellent experimental system for selectively obtaining primordial germ cells or germ stem cells from a transgenic non-human animal, establishing a cultured cell line derived therefrom, and assaying the ability of differentiating into germ cells as essential parts of the ES cell system. Also, the present invention enables germ cells and offspring thereof to be obtained from ES cell clones, the ability of which to contribute to the germ cell lineage has been lowered. According to the present invention, germ cells can be obtained in a relatively simple manner without complicated procedures such as gene introduction via homologous recombination.

The present application claims priority from Japanese patent application JP 2005-161279 filed on June 1, 2005, the contents of which are hereby incorporated by reference into this application. Also, the contents of the documents cited herein are incorporated by reference into this application.

### Brief Description of the Drawings

Fig. 1 shows the results of assaying promoter activity via luciferase assay using various promoter fragments of the Mvh gene.
Fig. 2 shows the results of assaying promoter activity via luciferase assay using a 3.0 Luc vector and, as host cells, various mouse cell lines.
Fig. 3 shows the cross-sectional image of the testis of the 3.0-GFP transgenic mouse.
Fig. 4 shows the results of the GFP fluorescent observation of the adult testis and the fetal testis of the 3.0-GFP-UTR transgenic mouse and the cross-sectional image of the tissue thereof *in vivo.*
Fig. 5 shows the results of GFP fluorescent observation of the testis of the 3.0-VASA-GFP transgenic mouse and the cross-sectional image of the tissue thereof.
Fig. 6 shows the results of visualizing germ cells using the BAC-Vasa-RFP gene.
Fig. 7 shows the results of identifying and culturing germ stem cells using BAC-Vasa-RFP.
Fig. 8 shows the structure of the 3.0-GFP vector.

## Claims

1. A method for obtaining germ cells which comprises recovering cells capable of differentiating germ cells from a transgenic non-human mammalian animal into which a recombinant expression vector comprising a marker gene under the control of a promoter sequence of a mammalian-derived Vasa homolog gene has been introduced by employing expression of said marker gene as an indicator.

2. A method for obtaining germ cells which comprises culturing ES cells or tissue stem cells into which a recombinant expression vector comprising a marker gene under the control of a promoter sequence of a mammalian-derived Vasa homolog gene has been introduced and recovering cells capable of differentiating germ cells by employing expression of said marker gene as an indicator.

3. The method according to claim 2, wherein the ES cells or tissue stem cells are mammalian- or avian-derived cell lines.

4. The method according to any of claims 1 to 3, wherein the promoter sequence of a mammalian-derived Vasa homolog gene comprises at least a nucleotide sequence comprising a region from a site 3 kb upstream of the transcription initiation point to the transcription initiation point.

5. The method according to any of claims 1 to 4, wherein the recombinant vector comprises, at a site downstream of the marker gene, a sequence of the 3'-untranslated region (UTR) of a mammalian-derived Vasa homolog gene ligated thereto.

6. The method according to any of claims 1 to 5, wherein the marker gene is a fluorescent protein-encoding gene, a drug-resistant gene, or a chemiluminescent enzyme gene.

7. The method according to any of claims 1 to 6, wherein the marker gene is a GFP, RFP, BFP, YFP, or lacZ gene.

8. The method according to any of claims 1 to 7, wherein the germ cells are germ stem cells.

9. A recombinant expression vector which comprises a marker gene under the control of a promoter sequence of a mammalian-derived Vasa homolog gene.

10. The recombinant expression vector according to claim 9, wherein the promoter sequence of a mammalian-derived Vasa homolog gene comprises at least a nucleotide sequence comprising a region from a site 3 kb upstream of the transcription initiation point to the transcription initiation point

11. The recombinant expression vector according to claim 9 or 10, wherein the recombinant vector comprises, at a site downstream of the marker gene, a sequence of the 3'-untranslated region (UTR) of a mammalian-derived Vasa homolog gene ligated thereto.

12. The recombinant expression vector according to any of claims 9 to 11, wherein the marker gene is a fluorescent protein-encoding gene, a drug-resistant gene, or a chemiluminescent enzyme gene.

13. The recombinant expression vector according to any of claims 9 to 12, wherein the marker gene is a GFP, RFP, BFP, YFP, or lacZ gene.

14. The recombinant expression vector according to any of claims 9 to 13, which is used for the method according to any of claims 1 to 8.

15. A cell which comprises the recombinant expression vector according to any of claims 9 to 14.

16. The cell according to claim 15, which is an ES cell or a tissue stem cell.

17. A transgenic non-human mammalian animal into which the recombinant expression vector according to any of claims 9 to 14 has been introduced.

18. A method for screening for a germ cell differentiation enhancing factor which comprises administering a test substance to a transgenic non-human mammalian animal into which a recombinant expression vector comprising a marker gene under the control of a promoter sequence of a mammalian-derived Vasa homolog gene has been introduced and selecting a test substance that induces expression of the marker gene.

19. A method for screening for a germ cell differentiation enhancing factor which comprises culturing an ES cell or a tissue stem cell into which a recombinant expression vector comprising a marker gene under the control of a promoter sequence of a mammalian-derived Vasa homolog gene has been introduced in the presence of a test substance and selecting a test substance that induces expression of the marker gene.

20. A germ cell differentiation enhancing factor which is obtained by the method according to claim 18 or 19.

21. A method for testing toxicity to germ cells which comprises administering a test substance to a transgenic non-human mammalian animal into which a recombinant expression vector comprising a marker gene under the control of a promoter sequence of a mammalian-derived Vasa homolog gene has been introduced and detecting a substance having toxicity to germ cells by employing expression of said marker gene as an indicator.

22. A method for testing toxicity to germ cells which comprises culturing an ES cell or tissue a stem cell into which a recombinant expression vector comprising a marker gene under the control of a promoter sequence of a mammalian-derived Vasa homolog gene has been introduced in the presence of a test substance and detecting a substance having toxicity to germ cells by employing the number of cells in which expression of the marker gene is induced as an indicator.


SEQUENCE LISTING

<110> Mitsubishi chemical corporation

<110> RIKEN

<120> EXPRESSION VECTOR COMPRISING PROMOTER SEQUENCE OF MAMMALIAN-DERIVED Vasa
HOMOLOG GENE AND USE THEREOF

<130> A61358A

<160> 10

<210> 1
<211> 2789
<212> DNA
<213> mouse

<400> 1

tctgcagtaa actgaatctc cagttttcac gttatgtaga tccatctcca tgaatctggc 60

ctggccttgt gaatttctgg taactgatta gttgaaagtt gcaaatataa ttgtttaact 120

tcttatgcta gaattcaaga agccttgcaa ctattaccct ggtcctttgg agcgatgtcc 180

ctgacaagtg tgtccagttg aaatgcagca tccttacaga gtctgactga gattgtccaa 240

caaccttcag agcctaagtc agcctcttgg aaactattca attgttctgg ccatcccagc 300

cactagatgc cagagtagag aagtcacctt ggatgttaaa caaactggta ttggaggttg 360

gggataaact cagtagtaca gagcttgcct ggcacgctca agtacctagg ttcaaactct 420

ggcaatgcca aaaaagaaaa aagttgatag caaactctca gatgattcaa gttctaatta 480

gcatttgaat ggaattgtat gagatggtct cctgagccct ctcaatccct gagagagaat 540

tttttttgta agtcaatata ttatgcaata gtttgttata tagttgtact ggctaatttg 600

ggtcaagctg gagttatcac agaaaaagga gcttcagttg gggaaatgcc tccatgagat 660

ccaactgtaa ggcattttct caattagtga tcaaggggga aaggcccctt gtgggtagga 720

ccatctctgg gctggtagtc ttggttctat aagagagcag gctgagcaag ccaggtgagg 780

caagccagta aggaacatcc ctcccatggc ctctgcatca gctcctgctc ctgacctgct 840

tgagttccag tcctgacttc ctttggtgat gaacagcaat gtggaaagtg taagctgaat 900

aaaccctttc ctccccaatt tgctcagtgg tcatgatgtt ttgtcctgga atagaaacct 960

tgactaagac aatagttata ggtaacaaga agagagctgt gaactcgtca tgtagttaag 1020

tgtttgctag atttcctatt gcatagttcc tattttctct ttatatttgg aaaaaaattg 1080

tggtaacata ccttagacac ttattgttgt tctcctccct gacctgcact caatgaattc 1140

tcagccaaac tctagtgatg tcctccccat tcaaaagctt caaccttcta gtctttattt 1200

taaaaaatgt tattattaca tttatttatt ctgtggtgtg tgtgtgtgag agagaggatg 1260

ggagcaggtg tgtgtgtgtg tgtcggtgtg tttgttaaat cagaacatga gtgtgtgtgt 1320

aagacagaga cggggtgagt gtgtgtgtgt gtgtgtgtgt gtgtgtgtgt cagtggataa 1380

gttgtagaag tcagttcttt ccttccctta ctcggacctc agggatcaaa ctcaagttct 1440

caggttgcca ggtacatgtc acctgtcttt gctttattat tatttagttc tggcttttga 1500

gcctaagtgt tatgttacat aggttgacct tgaattagct atgcactgag gtcctgatcc 1560
tcctgccttt atcttccaag taatggttac aggaatgggc cactggacct agcaagtgaa 1620
cctacctggt cactccaaaa atgcacagca aagaatatac gtttaaaaat aggttcattt 1680
taggaagttt gtccacattt taaatgacag ttctgttaaa gtatactgtg ttttgtcctt 1740
tgttaaatgt gacttttaaa agcaattcac cttaatagcc tgggcgacta cagtgctcac 1800
tgtataaatg ctagtgtgtt tttggtgctg aaacagaaag gtggctctag aaagctggag 1860
ttcctcatct ttaagttcca gactgagata tctacaactt cttcaaaaat ccagggaaag 1920
gaaagcgatg gaagtgaaaa taaaaacaag aacatgcttt acatatattt gattgtgatc 1980
cctttggcgg gtactaggaa aaccacggat ggaattttcc ttcttgaaag aggtgaggag 2040
caggcagaat gtgaacatct acttaatgag ctgaactggc cggtgccctc agaattgtaa 2100
acaggttcac cacaaatcca ggccttggca aacagaccaa gtcttcctct cttcggtttt 2160
ctttttacag actggctttc ttgacaactt caagatggag tctcatcctt gcccttttta 2220
tggagaggag aagcattgct tctagttggt tttagtagag gagtgaagtg catttctcag 2280
atacaaagag agcacttgag acgttcagac tcagaatggc caagcctggc actttgggag 2340
gtcaagagga ggctggaacg gctgggagag aaagcaatta gatgttccac ccctttggtt 2400
tttctccaga cagggtttct ctgtgtagcc ctgactgtcc tgaagtttgc tctgtagacc 2460
aggctggcct ccacccaggg atttgcctgc ctctgcctcc ccgagcccca gatttttatt 2520
tttatttatt tatatatcta ttttaacttt tgaatgaaca caatggaatt gatgagccct 2580
tggagagaga aacgggatgt cgtgcgtggc agccccgggg atcagctcac tcccacaggc 2640
ctcacaggcc atggagccaa gaggcctccc tgcctcggcc tcggcctcgg cctcaacaaa 2700
ggtggagaac gcgcaggccg tccgtccatg gggcgggaag tcgcgcgccg cggccgctga 2760
ttggctggcg ggcccggtcg cctgatgct 2789

<210> 2
<211> 2300
<212> DNA
<213> mouse

<400> 2

gattaaagca aacgaacata cttcaagtct gatagttttg atgcagagaa gaaagagttt 60
ttatttttaa aattttaaca gaagtgtgaa acctgatatt cttatatctc ctgttcttct 120
gttcttcctc ccaaccttta aaaaatagcc agcttcattg attagttatg tgaaatgctg 180
acgttacaac actgcagtta ctgatacaaa tggtgttagc tggaaatatt aaagcattct 240
tatatgtttt gcttatttct agtatattct tcagaaagtt aagacatgtt tcatgtccaa 300
gtgcttaagt cttagtatag tgtttatgat cctataaaac aagcaatagg atgtgatgtg 360
cttttgttta attattgggt ctaatttcta cttgatcctt taaaagaata gtgtatcagt 420
acaatgtaat aacatgattt tcatgaaaca gtagagactg aagcctttca aagttatttg 480
atttttagat catcagacat gtaatgaaaa tggttcagtt tgcaatgtga gctttgtact 540

tggtggtatg acaaatgttt gcttttataa tatacagatt ttccttgcaa ataaaagatg 600
aaacacattt ccccctaagt tttccaacag catttttcat ttttcttgta gcacattaca 660
gttcctacct ttcatatttg ggtattttat atcttgtgag atcagtatgg tgagcaagaa 720
gtgttagaca gatactaata tgtaaatcca tacaaatacc aagatattga catgaaaatt 780
agttttagaa tctaaaccac ttatcaacaa acatggtatt tcagtttcct cataactttt 840
tattcatgtt ttacacttga taaggaaaaa atgctgtcat tctcactcaa tcacagactt 900
acctacgcca tgagattttt tttccagata cttttttttc cttttattgg atattttctt 960
tatttacatt tcaaatatcc cccattccca atttcccctc tggaagccct ctatcccatc 1020
cctcctcccc ctgcttctat gagggtgctc ccctacccac ccacccactc cttcctcccc 1080
accctggcat tcccctacat tggggcatcg agccttcaca ggaccaatgg cctttcctcc 1140
cactgacaag gctgttctct gctacatatg tgactagagc catgggtcaa tccatacgta 1200
atctttgatt ggtggtttag tccctgggat ctgggggtgg gggcaggtct ggttggctga 1260
tattgttgtt cttcctatgg ggttgcaaac cccttcagct ccttcagtcc tttctctaac 1320
tcctccattg gggaccctgt ggttggctgt gagaattcac ctctgtattt gtgaggctct 1380
gacagagctt ctcaagagac agctatatca gggtcctgtc aacaaaatct tgctggcata 1440
tgcaatagtg tccgggtttg gtggttgttt atgggatgga tccccaggtg gggtagtttc 1500
tggatggtcc atccttccgt ctcagctccg aactttgtaa ctccttccat gggtattttg 1560
ttccccattc taagaaggaa cgaagaatct acactttggt cttccttctt gagtttcatg 1620
tgttttgcaa attgtatctt gggtattcta agtttctggg ctaatatcca cttatcagtg 1680
agtgcatatc aagtgacttc ttttgtgatt aggttgcctc actcaggatg atagcctcca 1740
gatatatcca tttgcccaag aatttcataa attcattatt tttaatagct gagtagtatt 1800
ctattgtgta aatgtaccac attttctggg tccttttcag cttctggcta ttataaatag 1860
ggctgctgtg aacatagtgg agcatgtgtc cttataacgg gttgggacat cttctggata 1920
tatgcccagg agaggtattg ctggatcctc cggtagtact atgtccaatt ttctgaggaa 1980
ccaccagact gtcccaccag caatggagga gtgttcctct ttctccacat cctcaccagc 2040
atctgctgtc acctgaggtt tttttttttt tttttttttt ttttttttaa tcttagccat 2100
tctgactggt gtgacataaa atctcagggt tgttttgatt ttcatttccc tgatgactaa 2160
ggatgttgaa agtttcttta ggtgcttctc ggccattcgg tattcctcag ctgggaattc 2220
ttttttagct ctttacccca ttttttaata gggttatgtg gttctctgga gtctaacttc 2280
tagagttctt tgtatatatt 2300

<210> 3
<211> 20
<212> DNA
<213> Artificial Sequence

<220>
<223> Description of Artificial Sequence: Synthetic DNA

<400> 3

gattaaagca aacgaacata                    20

<210> 4
<211> 21
<212> DNA
<213> Artificial Sequence

<220>
<223> Description of Artificial Sequence: Synthetic DNA

<400> 4

cccactgcac ataggactta t                  21

<210> 5
<211> 21
<212> DNA
<213> Artificial Sequence

<220>
<223> Description of Artificial Sequence: Synthetic DNA

<400> 5

gaagctatca tgggagatga a                  21

<210> 6
<211> 20
<212> DNA
<213> Artificial Sequence

<220>
<223> Description of Artificial Sequence: Synthetic DNA

<400> 6

ggcccatgat tcgtcatcaa                    20

<210> 7
<211> 21
<212> DNA
<213> Artificial Sequence

<220>
<223> Description of Artificial Sequence: Synthetic DNA

<400> 7

accatggtga gcaagggcga g                  21

<210> 8
<211> 20
<212> DNA
<213> Artificial Sequence

<220>
<223> Description of Artificial Sequence: Synthetic DNA

<400> 8

ctactcagac aatgcgatgc                    20

<210> 9
<211> 20
<212> DNA
<213> Artificial Sequence

<220>
<223> Description of Artificial Sequence: Synthetic DNA

<400> 9

tgtggaattg tgagcggata                    20

<210> 10
<211> 20
<212> DNA
<213> Artificial Sequence

<220>
<223> Description of Artificial Sequence: Synthetic DNA

<400> 10

gttttcccag tcacgacgtt                    20